# EUROPEAN PATENT APPLICATION

(11) **EP 3 903 873 A1**
(43) Date of publication of application: **03.11.2021**
(21) Application number: 19906112.8
(22) Date of filing: 06.08.2019
(51) Int. Cl.: A61M 39/06

(54) **HEMOSTATIC VALVE, SHEATH, AND CATHETER SHEATH ASSEMBLY**

(30) Priority: 24.12.2018 CN 201811585662; 24.12.2018 CN 201822176995 U
(71) Applicant: Hangzhou Endonom Medtech Co., Ltd, Hangzhou, Zhejiang 310051 (CN)
(72) Inventor: JIANG, Quanjie, Hangzhou, Zhejiang 310051 (CN); HUANG, Qiao, Hangzhou, Zhejiang 310051 (CN)
(74) Representative: Hamer, Christopher K.
(86) International application number: PCT/CN2019/099367
(87) International publication number: WO 2020/134092

(57) **Abstract**

A hemostatic valve, a sheath, and a catheter sheath assembly are provided. The hemostatic valve includes a valve body and a spool arranged in the valve body. The spool includes a spool main body and a cover body connected to a distal end of the spool main body. The spool main body defines an axial through hole therein. The cover body is operable to be opened or automatically closed relative to the spool main body to correspondingly deocclude or occlude the axial through hole. The hemostatic valve has an ideal sealing effect, can prevent blood leakage or air entering the human body, has high reliability, and is particularly suitable to be used with a dilator or other diagnostic and therapeutic device with a large diameter.

## Description

### TECHNICAL FIELD

The disclosure relates to the technical field of medical equipment, in particular to a hemostatic valve, a sheath, and a catheter sheath assembly.

### BACKGROUND

As an auxiliary introducer device for peripheral and intracardiac minimally-invasive interventional operations, a catheter sheath plays an important role in percutaneous coronary intervention, percutaneous interventional occlusion, interatrial septal puncture, and the like, which establishes a connection passage between human blood vessels and the external to assist a delivery system to deliver a diagnostic and/or therapeutic device to a target lesion location. A hemostatic valve is an indispensable component of the catheter sheath product, which is generally mounted at a proximal end of a sheath, and can prevent blood loss, reduce the amount of bleeding, prevent air entering the blood vessels to form air embolisms, and reduce patient complications.

In a related art, the hemostatic valve has the main structural forms as follows:
A Luer-taper opening type, a Luer taper with an axial opening is rotated to squeeze a basically cylindrical elastic member at a distal end of the Luer taper, so that a center bore diameter of the elastic member changes. When a dilator or other diagnostic and therapeutic device withdraws from the hemostatic valve, the Luer taper is rotated to move to the distal end to squeeze the elastic member until the center bore diameter of the elastic member is reduced to 0, thereby sealing a proximal end of the sheath. When the dilator or other diagnostic and therapeutic device is inserted in the sheath, the Luer taper is reversely rotated to move to the proximal end to moderately release the elastic member so that an outer circumference surface of the dilator or other diagnostic and therapeutic device is surrounded by the center bore of the elastic member, thereby achieving a sealing effect. However, the hemostatic valve that is of the Luer-taper opening type structure has obvious defects: when the dilator or other diagnostic and therapeutic device with a large diameter is required to thread in the hemostatic valve, an initial diameter of the center bore of the elastic member is relatively large, and the center bore of the elastic member may not be reduced to be fully closed by squeezing the Luer taper. Therefore this type of hemostatic valve has poor sealing effect and limited reliability in preventing blood loss, and still has a risk of blood leakage or air entering the human body when used with the dilator or other diagnostic and therapeutic device with a large diameter.

An "X"-shaped or "+"-shaped split type hemostatic valve, the hemostatic valve is provided with two penetration splits that are crisscrossed. In a natural state, the penetration splits are closed to close the proximal end of the sheath. When the dilator or other diagnostic and therapeutic device is threaded into the sheath through the penetration splits, the penetration splits are opened and fit an outer surface of the dilator or other diagnostic and therapeutic device to achieve a sealing effect. However, the "X"-shaped or "+"-shaped split type hemostatic valve also has obvious defects: when the dilator or other diagnostic and therapeutic device with a large diameter is threaded in the hemostatic valve, initial ends and tail ends of the penetration splits cannot fully fit the outer circumference surface of the dilator or other diagnostic and therapeutic device. In addition, after the dilator or other diagnostic and therapeutic device with a large diameter is frequently threaded in or withdrawn from the hemostatic valve, the penetration splits may not restore to a full closed state in the natural state, and thus, this type of hemostatic valve has poor sealing effect and limited reliability in preventing blood loss, and still has a risk of blood leakage or air entering the human body when used with the dilator or other diagnostic and therapeutic device with a large diameter.

### SUMMARY

The disclosure provides a hemostatic valve having an ideal sealing effect, capability of preventing blood leakage or air entering the human body, and high reliability, which is particularly suitable for being used with a dilator or other diagnostic and therapeutic device with a large diameter.

The disclosure further provides a sheath and a catheter sheath assembly both provided with the hemostatic valve, which have ideal sealing effects, can prevent a risk of blood leakage or air entering the human body, and can improve surgical safety and success rate.

In order to solve the above technical problems, the disclosure first provides a hemostatic valve, including a valve body and a spool arranged in the valve body. The spool includes a spool main body and a cover body connected to a distal end of the spool main body. The spool main body defines an axial through hole therein. The cover body is operable to be opened or automatically closed relative to the spool main body to correspondingly deocclude or occlude the axial through hole.

The disclosure further provides a sheath, including a tube body extended with an axial length and the hemostatic valve. The hemostatic valve is arranged at or close to a proximal end of the tube body.

The disclosure further provides a catheter sheath assembly, including the sheath and a dilator. The dilator is movably inserted in the tube body of the sheath and the axial through hole of the spool main body of the hemostatic valve.

The hemostatic valve, the sheath, and the catheter sheath assembly are provided in the disclosure. The spool of the hemostatic valve adapts a structure similar to a hatch door, including the spool main body and the cover body connected to the distal end of the spool main body. The cover body can be opened or automatically closed relative to the spool main body to correspondingly deocclude or occlude the axial through hole. When the dilator or other diagnostic and therapeutic device pushes the cover body along a direction toward the distal end to deocclude the axial through hole occluded by the cover body, sealing is formed between the dilator or other diagnostic and therapeutic device and the axial through hole. When the dilator or other diagnostic and therapeutic device is withdrawn out of the axial through hole, the cover body is immediately automatically reset under action of blood pressure to occlude the axial through hole to form sealing, thereby preventing blood leakage or air entering the human body and achieving an ideal sealing effect and high reliability. In addition, in comparison with the related art, the hemostatic valve that is of the structure similar to a hatch door breaks through the restriction on a diameter of the dilator or other diagnostic and therapeutic device, and is particularly suitable to be used with the dilator or other diagnostic and therapeutic device with a large diameter for sealing.

### BRIEF DESCRIPTION OF THE DRAWINGS

In order to describe the technical solutions in the disclosure more clearly, the accompanying drawings required to be used in implementations will be simply introduced below. It is apparent that the accompanying drawings in the following descriptions are only some implementations of the disclosure. Those of ordinary skill in the art may further obtain other apparent variations according to these accompanying drawings without creative work.
FIG. 1 is a three-dimensional structure schematic view of a catheter sheath assembly of a first implementation of the disclosure.
FIG. 2 is a three-dimensional structure schematic view of a hemostatic valve illustrated in FIG. 1.
FIG. 3 is a three-dimensional exploded view of FIG. 2.
FIG. 4 is a three-dimensional structure schematic view of a spool in FIG. 3 from another view.
FIG. 5 is a sectional view along a line V-V in FIG. 4.
FIG. 6 is a three-dimensional structure schematic view showing another state of the spool in FIG. 4.
FIG. 7 is a sectional view along a line VII-VII in FIG. 6.
FIG. 8 is a three-dimensional schematic view of the spool in another structure form in FIG. 3.
FIG. 9 is rear view of the spool in FIG. 8.
FIG. 10 is a sectional view along a line X-X in FIG. 8.
FIG. 11 is a sectional view of the spool in yet another structure form in FIG. 3.
FIG. 12 is a sectional view of the spool in still another structure form in FIG. 3.
FIG. 13 is a three-dimensional assembled schematic view of a valve housing in FIG. 3 from another view.
FIG. 14 is a sectional view along a line XIV-XIV in FIG. 13.
FIG. 15 is a three-dimensional assembled schematic view of the valve housing and a bonnet in FIG. 3.
FIG. 16 is a sectional view along a line XVI-XVI in FIG. 15.
FIG. 17 is a sectional view along a line XVII-XVII in FIG. 2.
FIG. 18 is a three-dimensional exploded view of a catheter sheath in FIG. 1.
FIG. 19 and FIG. 20 are schematic views showing an application process of the catheter sheath of the disclosure.
FIG. 21 is an enlarged view of a part XXI in FIG. 19.
FIG. 22 is an enlarged view of a part XXII in FIG. 20.
FIG. 23 is a three-dimensional exploded view of a hemostatic valve provided by a second implementation of the disclosure.
FIG. 24 is a structure schematic view of a catheter sheath assembly provided with the hemostatic valve in FIG. 23.
FIG. 25 is an enlarged view of a part XXV in FIG. 24.

### DETAILED DESCRIPTION

The technical solutions in implementations of the disclosure are clearly and completely described in the following in conjunction with the accompanying drawings of the disclosure. It is apparent that the described implementations are only part of the implementations of the disclosure, not all of the implementations. On the basis of the implementations of the disclosure, all other implementations obtained on the premise of no creative work of those of ordinary skill in the art shall fall within the scope of protection of the disclosure.

In addition, the following explanation of each implementation refers to illustration of an implementable specific implementation of the disclosure with reference to additional drawings. The direction terms mentioned in the disclosure, such as "up " , "down", "front", "back", "left", "right", "inner", "outer", and "side" are only the directions with reference to the additional drawings. Therefore, the used direction terms are intended to better and more clearly illustrate and understand the disclosure instead of indicating or implying that the device or element must have a specific orientation or must be constructed and operated in a specific orientation, and thus cannot be interpreted as limitation to the disclosure.

In order to describe structures of a hemostatic valve, a sheath, and a catheter sheath assembly more clearly, terms "proximal end" and "distal end" are defined in the disclosure as commonly used terms in the field of interventional medical treatment. Specifically, "distal end" indicates one end away from an operator during a surgical operation, and "proximal end" indicates one end close to the operator during the surgical operation. Unless otherwise defined, all technical and scientific terms used in the disclosure have the same meaning as commonly understood by those skilled in the art of the disclosure. The terms used in the specification of the disclosure herein are only for the purpose of describing the specific implementations, and are not intended to limit the disclosure.

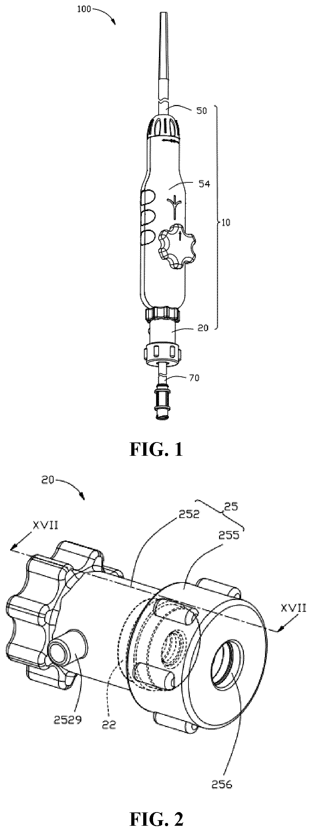

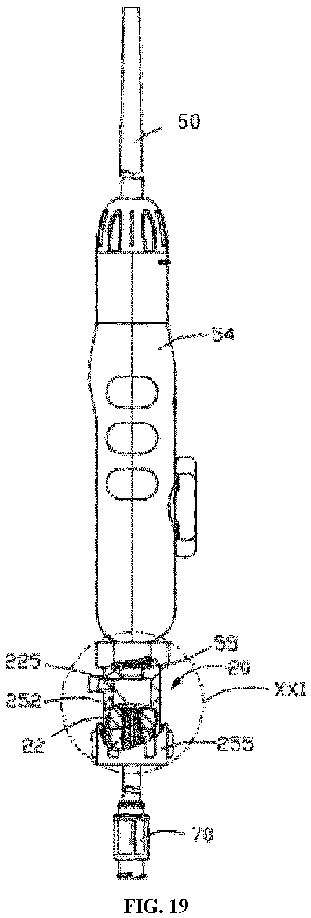

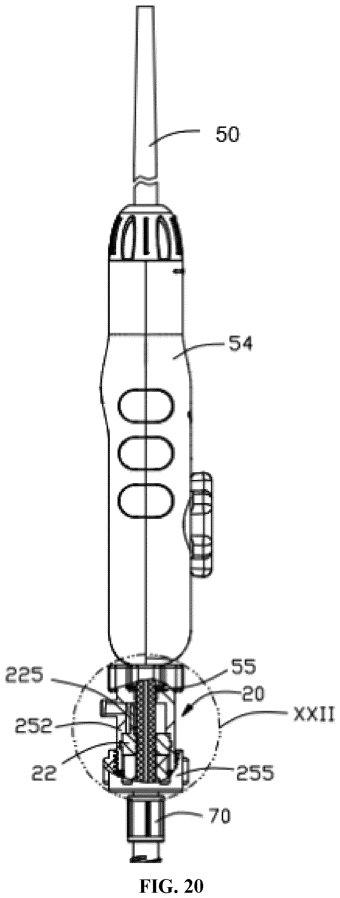

Referring to FIG. 1 and FIGS. 18-20, a catheter sheath assembly 100 is provided. The catheter sheath assembly 100 includes a sheath 10 and a dilator 70. The sheath 10 includes a hemostatic valve 20 and a tube body 50 extended with an axial length. The hemostatic valve 20 is arranged at or close to a proximal end of the tube body 50. Further, the sheath 10 also includes a handle 54 arranged at the proximal end of the tube body 50. The hemostatic valve 20 is detachably connected to a proximal end of the handle 54. The dilator 70 can be movably inserted in the hemostatic valve 20, the handle 54, and the tube body 50 in sequence. The tube body 50 is bendable or non-bendable. The handle 54 can apply an operation to the tube body 50. For example, the handle 54 can bend the distal end of the tube body 50. It can be understood that in other implementations, the hemostatic valve 20 could detachably connect to a distal end of the handle 54, and the dilator 70 can be movably inserted in the handle 54, the hemostatic valve 20, and the tube body 50 in sequence.

Referring to FIGS. 2 to 7, the hemostatic valve 20 includes a spool 22 and a valve body 25, and the spool 22 is arranged in the valve body 25. The spool 22 includes a spool main body 221 and a cover body 225 connected to a distal end of the spool main body 221. An axial through hole 220 is formed in the spool main body 221 for threading of the dilator 70 or other diagnostic and therapeutic device. The cover body 225 is operable to be opened or automatically closed relative to the spool main body 221 to correspondingly deocclude or occlude the axial through hole 220. The dilator 70 is movably threaded in the axial through hole 220 of the spool main body 221 of the hemostatic valve 20 and the tube body 50. When the dilator 70 pushes the cover body 225 of the spool main body 221 along a direction toward the distal end, the axial through hole 220 occluded by the cover body 225 will be deoccluded. When the dilator 70 is withdrawn, the cover body 225 automatically will reset to occlude the axial through hole 220.

The hemostatic valve 20, the sheath 10, and the catheter sheath assembly 100 are provided. The hemostatic valve 20 includes the spool 221 and the cover body 225. The spool 221 defines the axial through hole 220 therein for inserting the dilator 70. The cover body 225 can be opened or automatically closed relative to the spool main body 221 to correspondingly deocclude (i.e., expose) or occlude (i.e., close) the axial through hole 220, which is similar to a hatch door structure. When the dilator 70 or other diagnostic and therapeutic device pushes the cover body 225 along a direction toward the distal end to deocclude the axial through hole 220 occluded by the cover body 225, sealing is formed between the dilator 70 or other diagnostic and therapeutic device and the axial through hole 220. When the dilator 70 or other diagnostic and therapeutic device is removed out of the axial through hole 220, the cover body 225 is immediately automatically reset under action of blood pressure to occlude the axial through hole 220 to form sealing, thereby preventing blood leakage or air entering the human body during the entire operation, achieving an ideal sealing effect and high sealing reliability, and facilitating improvement of surgical safety and success rate. In addition, in comparison with the related hemostatic valve, the hemostatic valve 20 that is of the structure similar to a hatch door breaks through the restriction on a diameter of the dilator or other diagnostic and therapeutic device, and is particularly suitable to be used with the dilator or other diagnostic and therapeutic device with a large diameter for sealing.

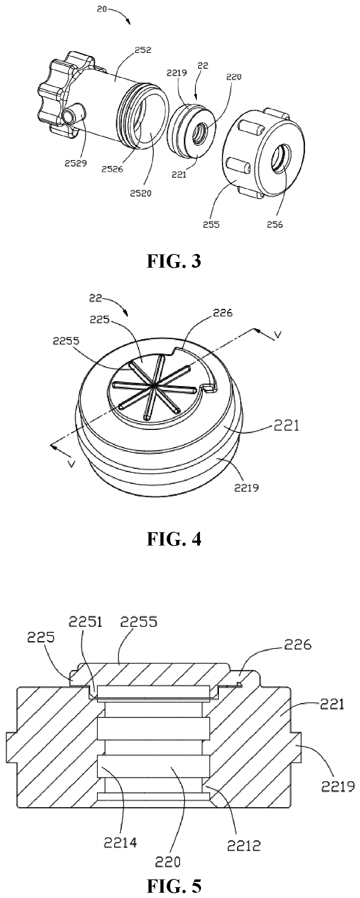

Referring to FIGS. 4 to 7, the spool 22 is made of an elastic and waterproof material. In an implementation, the spool 22 may be made of silicone, elastic rubber, elastic plastics, and other elastic and waterproof materials. In another implementation, the spool 22 may be made of a material such as a polystyrene elastomer, a polyethylene elastomer, a polyurethane elastomer, silicone rubber or a polyisoprene rubber elastomer. In this implementation, the spool 22 is made of silicone. The spool 22 may be shaped as a cylinder, a rectangular body, a kidney, a polygonal body or an irregular body, as long as the spool 22 can be hermetically accommodated in the valve body 25. In this implementation, the spool 22 is shaped as a cylinder.

As shown in FIG. 4 and FIG. 5, the distal end of the spool 22 is provided with the cover body 225. In this implementation, the cover body 225 may be rotationally connected to a position, close to the axial through hole 220, on a distal surface of the spool main body 22. The cover body 225 is similar to a hatch door, may rotate relative to the spool main body 221 to be away from the axial through hole 220 when subjected to a thrust toward the distal end, so as to deocclude the axial through hole 220 forming communication. In an implementation, when a distal end of the dilator 70 passes through the axial through hole 220 and pushes a proximal surface, facing the axial through hole 220, of the cover body 225, so that the cover body 225 is pivoted to be detached from the axial through hole 220 to release the cover body 225 occluding the axial through hole 220. This moment, a connection portion between the cover body 225 and the spool main body 221 elastically deforms. After the dilator 70 is withdrawn, the thrust that pushes the cover body 225 toward the distal end disappears, the cover body 225 may be reset under action of its elastic recovery to occlude the axial through hole 220 again, so that the axial through hole 220 is in a closed state. In addition, blood may press the cover body 225 toward the proximal end, so that the cover body 225 is closed more quickly and reliably. Only when the thrust, toward the distal end, on the cover body 225 is greater than a pressure of blood on the cover body 225, the cover body 225 will be opened by pushing. In an implementation, after the dilator 70 is withdrawn from the axial through hole 220 to release the pushing to the cover body 225, the connecting portion between the cover body 225 and the spool main body 221 is elastically reset to reset the cover body 225 so as to occlude the axial through hole 220.

The axial through hole 220 axially extends along the tube body 50 and through the proximal surface and the distal surface of the spool main body 221. In an implementation, the axial through hole 220 is formed in a middle position of the spool main body 221 along an axial direction of the spool main body 221. Multiple inner flanges 2212 are arranged at intervals on an inner circumference wall of the axial through hole 220 in the spool main body 221. Each inner flange 2212 is circumferentially arranged in a circle along the inner circumference wall of the axial through hole 220. Each two adjacent inner flanges 2212 cooperate to encircle a circular groove 2214 therebetween. The inner flanges 2212 are made of elastic waterproof materials. When being squeezed by an outer circumference wall of the dilator 70, each inner flange 2212 may elastically deform to be received in the corresponding circular groove(s) 2214. Thus, an inside diameter value of the axial through hole 220 determines a maximum diameter of a sheath core (that is, a dilator or other diagnostic and therapeutic device) that can pass through the axial through hole 220, and an inside diameter value of each inner flange 2212 determines a magnitude of interference between the inner flange 2212 and the sheath core and a minimum diameter of the sheath core that can pass through the axial through hole 220. Theoretically, the greater the magnitude of interference, the better the sealing effect, but at the same time, the excessive magnitude of interference makes the sheath core more resistant during an insertion process. The inside diameter values of the axial through hole 220 and the inner flanges 2212 may be adaptively designed according to a diameter range of the sheath core actually to be inserted, so that the hemostatic valve 20 can achieve a good sealing effect even for the sheath core with a large diameter. The hemostatic valve 20 in the disclosure can be used with a large sheath of 24F-15F. In an implementation, the inside diameter value of each inner flange 2212 is 5 mm to 10 mm less than that of the axial through hole 220. When the dilator 70 of a diameter greater than the inside diameter value of the inner flange 2212 is inserted in the axial through hole 220, a sealing effect is achieved by interference fit between the inner flange 2212 and the dilator 70, which can prevent blood from permeating between the dilator 70 and an inner circumference surface of the axial through hole 220 when the cover body 225 is opened. The number of the inner flanges 2212 is not limited, and is preferably 1-3. In this implementation, the number of the inner flanges 2212 is 3.

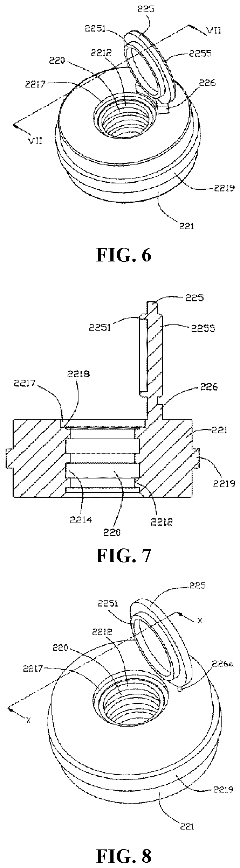

As shown in FIG. 6 and FIG. 7, one of the distal end of the spool main body 221 or a proximal end of the cover body 225 is provided with a first spigot structure, and the other is provided with a second spigot structure matching the first spigot structure, and the first spigot structure and the second spigot structure are jointed with each other to form a spigot, so that the cover body 225 closely and hermetically covers the axial through hole 220 of the spool main body 221.

In this implementation, the distal surface of the spool main body 221 defines a counter bore 2217 that is coaxial with the axial through hole 220 and serves as the first spigot structure, a diameter of the counter bore 2217 is greater than that of the axial through hole 220 and less than an outside diameter of the cover body 225, the proximal end of the cover body 225 is provided with a circular flange 2251 that matches the counter bore 2217 and serves as the second spigot structure, and the circular flange 2251 is used to be embedded in the counter bore 2217 to form a spigot. When the cover body 225 covers the spool main body 221, the circular flange 2251 is clamped in the counter bore 2217. It can be understood that in the other implementations, the circular flange may be arranged on the distal surface of the spool main body 221 while the counter bore may be arranged at the proximal end of the cover body 225.

In this implementation, the counter bore 2217 expands along the edge of the axial through hole 220, and is communicated to the axial through hole 220. Because an inside diameter value of the counter bore 2217 is greater than that of the axial through hole 220, the spool main body 221 is provided with a counter surface 2218 formed between the counter bore 2217 and the axial through hole 220. Because the inside diameter value of the counter bore 2217 is less than the outside diameter of the cover body 225, the cover body 225 can be prevented from falling into the counter bore 2217. In an implementation, the inside diameter value of the counter bore 2217 is about two thirds of the diameter of the cover body 225. An outside diameter of the circular flange 2251 is equal to or slightly greater than the inside diameter value of the counter bore 2217. When the cover body 225 covers the spool main body 221, the circular flange 2251 can be closely clamped in the counter bore 2217, and is abutted against the counter surface 2218, to close the axial through hole 220 and prevent the cover body 225 from falling into the axial through hole 220. When the cover body 225 covers the spool main body 221, the cover body 225 can effectively seal the distal end of the axial through hole 220. When the hemostatic valve 20 is applied to the sheath 10 and the catheter sheath assembly 100, blood pressure in the tube body 50 may further press the cover body 225 toward the proximal end after the axial through hole 220 is closed by the cover body 225, and thus, the sealing reliability of the hemostatic valve 20 can be further improved while the sealing effect can be improved.

In other implementations, the second spigot structure may also be a plate that can be accommodated in the counter bore 2217. That is, the plate may be a circular plate which is protrusively arranged on one side, facing the axial through hole 220, of the proximal end of the cover body 225. A diameter of the circular plate is equal to or slightly greater than the inside diameter value of the counter bore 2217, so that the circular plate can be hermetically accommodated in the counter bore 2217, and a proximal surface of the circular plate can be abutted against the counter surface 2218.

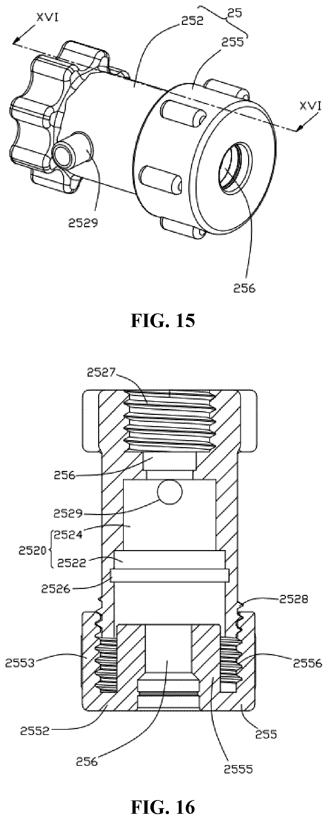

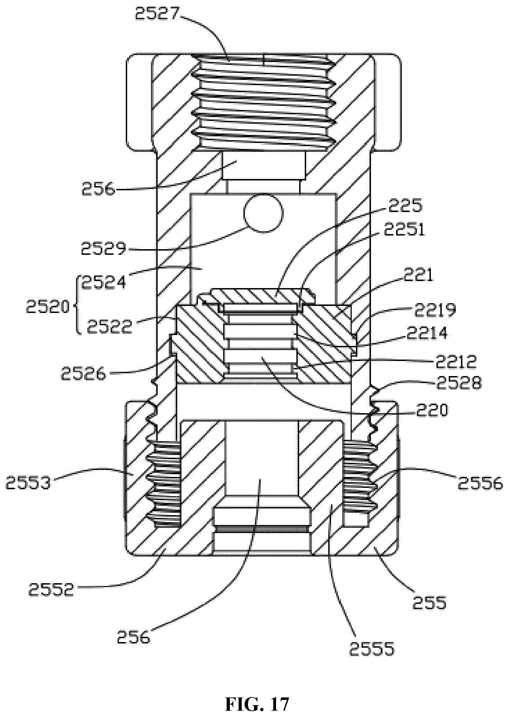

Referring to FIGS. 4-7, FIG. 16, and FIG. 17, an outer circumference surface of the spool 22 and the valve body 25 are in hermetical contact. In an implementation, the outer circumference surface of the spool 22 and the valve body 25 are positioned by means of matching between a positioning clamp ring and a positioning clamp groove. In an implementation, the outer circumference surface of the spool main body 221 is provided with positioning clamp rings 2219, and positioning clamp grooves 2526 matching the positioning clamp rings 2219 are formed in the valve body 25. When the spool 22 is accommodated in the valve body 25, the positioning clamp rings 2219 are clamped in the corresponding positioning clamp grooves 2526 to prevent the spool main body 221 from axially sliding. In addition, the positioning clamp rings 2219 and the positioning clamp grooves 2526 are respectively in interference fit radially to prevent blood from leaking between the spool 22 and the valve body 25. The number of the positioning clamp rings 2219 is not limited, is preferably 1 or 2. In this implementation, one positioning clamp ring 2219 is provided, and is circumferentially arranged in a continuous circle on the outer circumference surface of the spool main body 221.

As shown in FIG. 4, the cover body 225 is provided with multiple reinforcing ribs 2255 for increasing a strength of the cover body. In an implementation, the multiple reinforcing ribs 2255 are arranged on a distal surface and/or a proximal surface of the cover body 225 to increase the strength of the cover body 225, thereby preventing the cover body 225 from buckling deformation toward the distal end under high blood pressure to result in blood leakage. Further, the multiple reinforcing ribs 2255 may be uniformly distributed in cross respectively through the center of the cover body 225, or crisscross distributed, or distributed in other manners. In this implementation, the reinforcing ribs 2255 are arranged on the distal surface of the cover body 225 and are uniformly distributed in cross respectively through the center of the cover body 225.

The cover body 225 is connected to the spool main body 221 through an elastic connecting part 226. In this implementation, the connecting part 226 is an elastic connecting piece connected between the cover body 225 and the spool main body 221 and extending along a periphery of the axial through hole 220, which is substantially an arc entity. The connecting part 226 forces the cover body 225 to automatically occlude the axial through hole 220 in a natural state. The natural state refers to a state that the cover body 225 is not subjected to an external force. After the cover body 225 is opened, the connecting part 226 is elastically reset to drive the cover body 225 to be automatically closed, and under pressing of blood to the cover body 225, the cover body 225 may occlude the axial through hole 220 more rapidly and closely. As shown in FIG. 5, when the cover body 225 closes the axial through hole 220, the cross section of the connecting part 226 is L-shaped or circular-arc-shaped. In this implementation, the spool main body 221, the cover body 225, and the connecting part 226 are integrally molded by elastic waterproof materials.

Referring to FIGS. 8 to 10, another structural form of the spool in the disclosure is similar to a structure of the spool in the first implementation, and the difference between the two lies in: in the spool that is of another structural form, a connecting part 226a between the cover body 225 and the spool main body 221 is multiple elastic connecting rods, and the elastic connecting rods can be elastically reset to automatically drive the cover body 225 to occlude the axial through hole 220 of the spool main body 221. The elastic connecting rods are arranged at intervals. In an implementation, the elastic connecting rods are arranged at intervals along the periphery of the axial through hole 220.

The connecting part 226a may serve as an independent component connected between the cover body 225 and the spool main body 221. The connecting part 226a may also be made of an elastic waterproof material integrally molded with the spool main body 221 and the cover body 225.

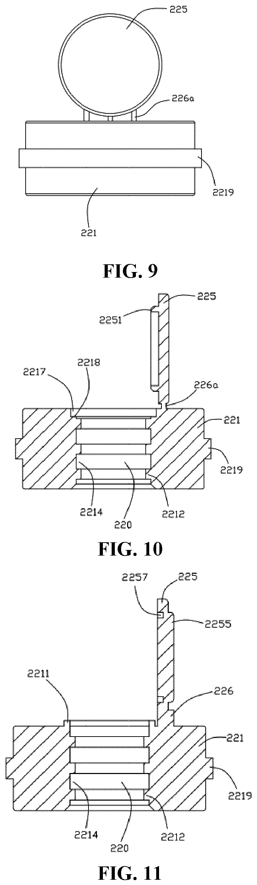

Referring to FIG. 11, yet another structural form of the spool in the disclosure is similar to the structure of the spool in the first implementation, and the difference between the two lies in: in the spool that is of yet another structural form, the distal surface of the spool main body 221 is protrusively provided with a circular flange 2211 serving as a first spigot structure. The cover body 225 defines a positioning circular groove 2257 matching the circular flange 2211 as a second spigot structure. When the cover body 225 occludes the axial through hole 220, the circular flange 2211 is clamped in the positioning circular groove 2257.

In yet another structure of the spool, the circular flange 2211 is protrusively arranged on the distal surface of the spool main body 221, and surrounds the edge of the axial through hole 220 by a circle, and the positioning circular groove 2257 is formed on the distal surface of the cover body 225. When the cover body 225 closes the axial through hole 220, the circular flange 2211 is clamped in the positioning circular groove 2257.

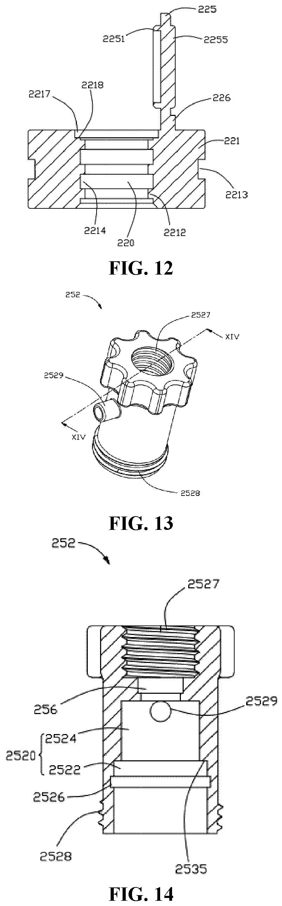

Referring to FIG. 12, still another structure of the spool in the disclosure is similar to the structure in the first implementation, and the difference between the two lies in: in the spool that is of still another structure form, at least one positioning clamp groove 2213 is circumferentially formed on the outer circumference surface of the spool main body 221, and positioning clamp rings corresponding to the positioning clamp grooves 2213 are arranged in the valve body 25. When the spool 22 is accommodated in the valve body 25, the positioning clamp rings of the valve body 25 are clamped in the corresponding positioning clamp grooves 2213 to prevent the spool main body 221 from axial sliding. The positioning clamp rings and the positioning clamp grooves 2213 are respectively in interference fit radially to prevent blood from leaking between the spool 22 and the valve body 25. The number of the positioning clamp grooves 2213 is not limited, and is preferably 1 or 2. In this implementation, the positioning clamp groove 2213 is circumferentially arranged in a continuous circle along the outer circumference surface of the spool main body 221.

Referring to FIGS. 13 to 16, the valve body 25 may be made of a high-polymer material or a metal material. In this implementation, the valve body 25 is made of a transparent PC material. The valve body 25 includes a valve housing 252 and a bonnet 255 connected to the valve housing 252. In an implementation, the bonnet 255 is detachably connected to a proximal end of the valve housing 252. The valve body 25 defines a cavity 256 extending along an axial direction of the tube body 50. The cavity 256 extends through the valve housing 252 and the bonnet 255. The valve housing 252 is substantially tubular, which may be shaped as a cylindrical tube, a rectangular tube, a polygonal tube, or a tube in other shape. In this implementation, the valve housing 252 is a cylindrical tube. The cavity 256 axially extends through a distal surface and a proximal surface of the valve housing 252. An accommodating space 2520 that is coaxial with the cavity 256 is formed in the valve housing 252. An inside diameter valve of the accommodating space 2520 is greater than that of the cavity 256. The accommodating space 2520 is used to accommodate the valve body 22.

In an implementation, the accommodating space 2520 has a positioning section 2522 and an avoiding section 2524 axially communicated to the positioning section 2522. The positioning section 2522 is used to position the spool main body 221, and the avoiding section 2524 is used to provide a space for opening of the cover body 225 of the spool 22. In an implementation, the positioning section 2522 is coaxial with the avoiding section 2524. The positioning section 2522 and the avoiding section 2524 are arranged sequentially along a direction from the proximal end to the distal end of the spool main body 221. That is, the positioning section 2522 is arranged at the proximal end of the valve housing 252, and extends through the proximal surface of the valve housing 252. The avoiding section 2524 is arranged at the distal end of the positioning section 2522. An inside diameter value of the positioning section 2522 is equal to or slightly less than the outside diameter of the spool main body 221, so that the outer circumference surface of the spool main body 221 may hermetically fit an inner circumference surface of the positioning section 2522 of the accommodating space 2520. The inside diameter value of the positioning section 2522 is greater than that of the avoiding section 2524, so that the valve housing 252 is provided with a positioning surface 2535 formed between the positioning section 2522 and the avoiding section 2524. When the spool main body 221 is accommodated in the positioning section 2522, the distal end of the spool main body 221 can be abutted against the positioning surface 2535. An inside diameter value of the avoiding section 2524 is greater than the outside diameter of the cover body 225 of the spool 22, and an axial extension length of the avoiding section 2524 is greater than the outside diameter of the cover body 225, so that the cover body 225 can be completely accommodated in the avoiding section 2524 when being opened.

The outer circumference surface of the spool 22 and the inner circumference surface of the positioning section 2522 of the accommodating space 2520 are positioned by means of clamping between positioning clamp rings and positioning clamp grooves. In this implementation, at least one positioning clamp groove 2526 that is annular is arranged on an inner wall surface of the positioning section 2522 of the accommodating space 2520 of the spool main body 221. The at least one positioning clamp groove 2526 is circumferentially arranged in a circle around the positioning section 2522. An inside diameter value of the positioning clamp groove 2526 is greater than that of the positioning section 2522. The positioning clamp groove 2526 is used to clamp the positioning clamp ring 2219 of the spool main body 221, so that the spool 22 can be positioned in the valve housing 252 and cannot axially move. Further, the inside diameter value of the positioning clamp groove 2526 is slightly less than an outside diameter of the positioning clamp ring 2219 of the spool main body 221, and an axial extension length of the positioning clamp groove 2526 is greater than that of the positioning clamp ring 2219 of the spool main body 221. When the spool 22 is accommodated in the accommodating space 2520, the positioning clamp ring 2219 of the spool main body 221 and the positioning clamp groove 2526 are in interference fit radially to prevent blood from leaking between the outer circumference surface of the spool 22 and the inner circumference surface of the valve housing 252. In addition, an axial deformation space is reserved between the positioning clamp ring 2219 and the positioning clamp groove 2526. When the spool main body 221 is squeezed, the positioning clamp ring 2219 can fill the deformation space, thereby radially sealing the spool 22 and the valve body 25.

In can be understood that, in other implementations, at least one positioning clamp ring that is annular is protrusively arranged on an inner wall surface of the positioning section 2522 of the accommodating space 2520 of the valve housing 252. The at least one positioning clamp ring is circumferentially arranged in a circle around the positioning section 2522. The inside diameter value of the positioning clamp ring is less than that of the positioning section 2522. The positioning clamp ring is used to be clamped in the positioning clamp groove 2213 of the spool main body 221 as shown in FIG. 12, thereby preventing the spool 22 from axially moving. In an implementation, the inside diameter value of the positioning clamp ring is slightly less than that of the positioning clamp groove 2213, so that the positioning clamp ring and the positioning clamp groove 2213 can achieve interference fit radially to prevent blood from leaking between the outer circumference surface of the spool 22 and inner circumference surface of the valve housing 252.

In an implementation, an internal thread 2527 is formed on an inner wall surface of the cavity 256 at the distal end of the valve housing 252 and is used for connecting the valve body 25 to the handle 54. Multiple anti-slip strips are arranged on the outer circumference surface of the distal end of the valve housing 252, which facilitates gripping. An external thread 2528 is arranged on the outer circumference surface of the proximal end of the valve housing 252 and used for connecting the bonnet 255. The valve housing 252 defines a through hole 2529 radially extends to be in communication with the cavity 256. In an implementation, the through hole 2529 extends through the avoiding section 2524 of the accommodating space 2520. The through hole 2529 is used for connecting a three-way valve arranged outside the valve body 25.

The bonnet 255 may be shaped as a cylinder, a rectangle, a polygon, or in other shapes. In an implementation, the bonnet 255 is cylindrical. The bonnet 255 includes a circular proximal plate 2552, a ring-shaped side plate 2553 extending from the peripheral edge of the proximal plate 2552, and a squeezing block 2555 that is protrusively arranged in the middle of the proximal plate 2552 and protrudes to the distal end. A gap is reserved between the squeezing block 2555 and the side plate 2553. An internal thread 2556 is formed on an inner circumference surface of the side plate 2553 and matches an external thread 2528 arranged at the proximal end of the valve housing 252, thereby facilitating connecting the bonnet 255 to the proximal end of the valve housing 252. The cavity 256 axially extends through the squeezing block 2555 and the proximal plate 2552.

Referring to FIG. 2, FIG. 3, and FIG. 17, during assembling of the hemostatic valve 20, the distal end, provided with the cover body 225, of the spool 22 is mounted in the accommodating space 2520 from the proximal end of the valve housing 252, until the spool main body 221 of the spool 22 is accommodated in the positioning section 2522, and the cover body 225 is accommodated in the avoiding section 2524. Here, the positioning clamp ring 2219 is clamped in the positioning clamp groove 2526. The outer circumference surface of the spool main body 221 is in close contact with the inner circumference surface of the positioning section 2522. The positioning clamp ring 2219 is in interference fit with the positioning clamp groove 2526. The cover body 225 occludes the axial through hole 220 of the spool main body 221. The circular flange 2251 is clamped in the counter bore 2217, so that the spool 22 and the valve housing 252 are hermetically connected. And then, the bonnet 255 is connected to the proximal end of the valve housing 252 by screwing.

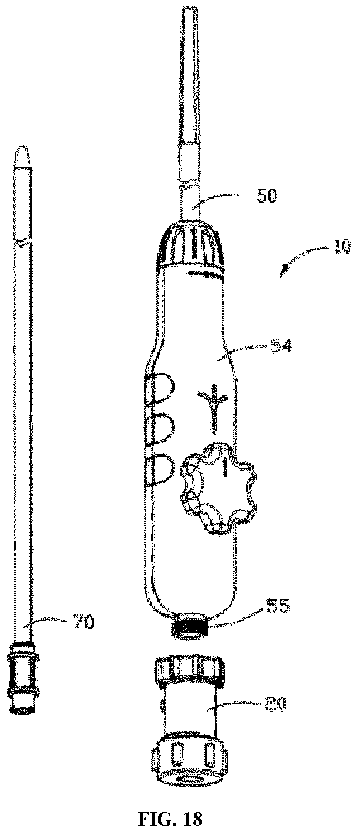

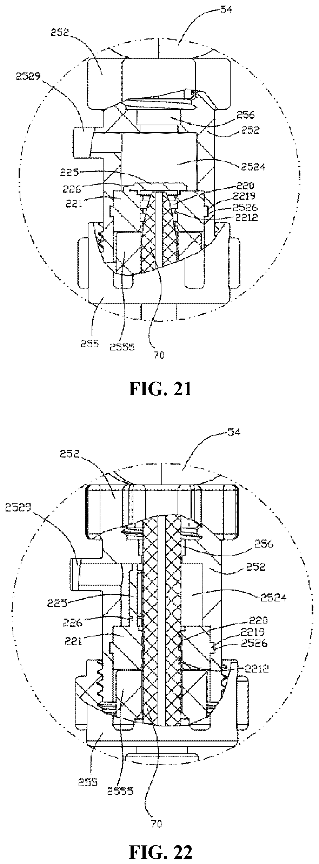

Referring to FIGS. 18 to 22, taking interatrial septal puncture as an example, the catheter sheath assembly 100 is used as follow. The distal end of the hemostatic valve 20 is connected to the proximal end of the handle 54 of the sheath 10. In an implementation, the distal end of the handle 54 is provided with a joint tube 55 with an external thread, and the internal thread 2527 at the distal end of the valve housing 252 of the hemostatic valve 20 is connected to the external thread of the joint tube 55 by screwing. Then, vascular puncture is performed. The distal end of the tube body 50 of the sheath 10 is delivered to the neighboring interatrial septum. Here, the cover body 255 of the hemostatic valve 20 occludes the axial through hole 220, and closely fits the spool main body 221, under action of blood pressure, to prevent blood leakage and air entering the human body. The dilator 70 is then inserted in the sheath 10. In an implementation, when the distal end of the dilator 70 pushes the proximal surface of the cover body 255 of the spool 22, the cover body 225 leaves from the spool main body 221, until the cover body 225 is fully opened by the distal end of the dilator 70. The cover body 255 is accommodated in the avoiding section 2524 of the accommodating space 2520. The connecting part 226 between the cover body 225 and the spool main body 221 elastically deforms. The dilator 70 may be continuously pushed to the distal end to reach a specified position. During this process, the inner flanges 2212 in the axial through hole 220 are in interference fit with the dilator 70 to achieve sealing, thereby preventing blood leakage and air entering the human body. After that, a puncture needle is inserted in the dilator 70 to perform interatrial septal puncture. When the puncture is completed, the puncture needle is withdrawn into the dilator 70 and removed together with the dilator 70. During withdrawing of the dilator 70, when the distal end of the dilator 70 is withdrawn into the axial through hole 220 of the spool main body 221, the pushing from the dilator 70 to the cover body 225 is released, and under co-action of an elastic reset force of the connecting part 226 between the cover body 225 and the spool main body 221 and compression from blood pressure, the cover body 225 is instantly restored to the initial position to fit the spool main body 221. In other words, the cover body 225 occludes the axial through hole 220 of the spool main body 221 for sealing, and the circular flange 2251 is clamped in the counter bore 2217. Therefore, blood leakage and air entering the human body may be avoided during back withdrawing of the dilator 70, and the hemostatic valve 20 achieves a reliable and good sealing effect during the entire operation. In the disclosure, for the dilator 70 with large outside diameter, related dimensions of the cover body 225, the axial through hole 220, and the inner flange 2212 are adaptively set, so that the hemostatic valve 20 can ensure a good sealing effect, breaks through the restriction on the diameter of the dilator or other diagnostic and therapeutic device, and accordingly is particularly suitable to be used with the dilator 70 or other diagnostic and therapeutic device with a large diameter for sealing. In addition, with arrangement of the cover body 225 that can be deoccluded and occluded, and the multiple inner flanges 2212 in the axial through hole 220 of the spool main body 221, a resistance force to the dilator 70 when being pushed may be small.

After the interatrial septal puncture, the inner cavity of the tube body 50 and the axial through hole 220 of the hemostatic valve 20 may be used for delivering other diagnostic and therapeutic device if necessary.

The hemostatic valve 20 in the disclosure can achieve reliable sealing throughout the entire process, have an excellent sealing effect, prevent the risks of blood leakage and air entering the human body, and improve surgical safety and success rate. Except for pushing or back withdrawing the dilator 70 or other diagnostic and therapeutic device, extra operation to the hemostatic valve 20 is not needed. Thus, the hemostatic valve 20 is easy to operate and convenient to use.

In other implementations, the hemostatic valve 20 and the sheath 10 may be connected by means of clamping, gluing, welding, or the like, as long as the cavity 256 of the hemostatic valve 20 is communicated to the sheath 10.

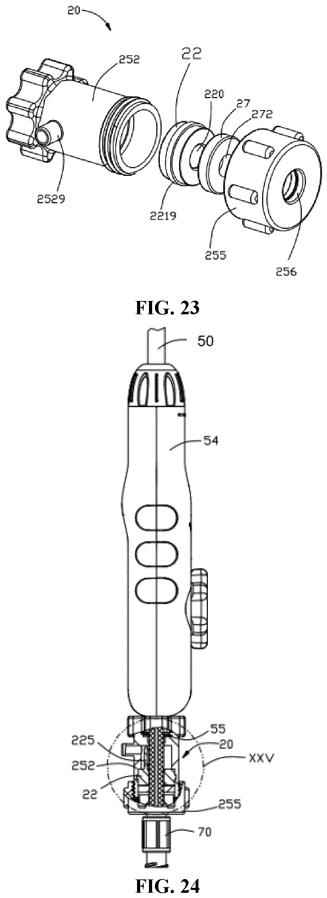

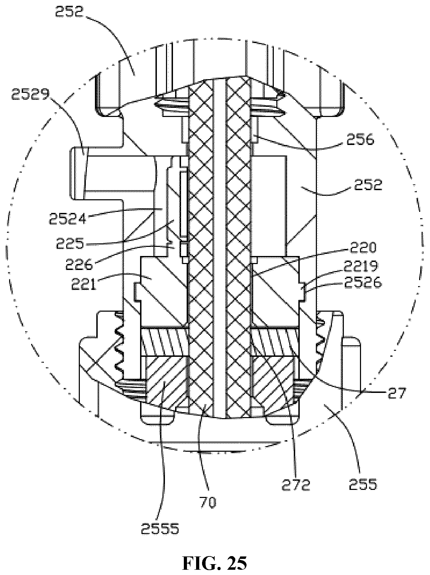

Referring to FIGS. 23 to 25, a structure of the catheter sheath assembly provided in a second implementation of the disclosure is similar to the structure provided in the first implementation, and the difference between the two lies in: in the second implementation, the hemostatic valve 20 further includes an elastic gasket 27 sandwiched between the spool 22 and the bonnet 255. The elastic gasket 27 can be squeezed to deform by axially moving the bonnet 255. In an implementation, the elastic gasket 27 is axially provided with a through hole 272 communicated to the axial through hole 220 of the spool main body 22. An inside diameter value of the through hole 272 is equal to or slightly less than an outside diameter of the dilator 70, so that an inner circumference surface of the through hole 272 of the elastic gasket 27 and an outer circumference surface of the dilator 70 are in interference fit to achieve sealing for threading of the dilator 70. The bonnet 255 is driven to axially move to the distal end or the proximal end to clamp or release the elastic gasket 27, so that the elastic gasket 27 deforms to control decrease or increase of a diameter of the through hole 272 in the elastic gasket 27. In an implementation, the bonnet 255 is screwed on or screwed off to drive the squeezing block 2555 thereof to clamp or release the elastic gasket 27, so that the elastic gasket 27 deforms to control increase or decrease of the inside diameter value of the through hole 272 of the elastic gasket 27 to adapt to the dilator 70 with different outside diameter.

The elastic gasket 27 may be cylindrical, polygonal, or the like. In an implementation, the elastic gasket 27 is cylindrical. The elastic gasket 27 is made of a material such as silicone, elastic rubber, and elastic plastics. In this implementation, the elastic gasket 27 is made of silicone.

In this implementation, since the hemostatic valve 20 is provided with the cover body 225, the cover body 225 automatically occludes the axial through hole 220 after the dilator 70 is withdrawn, and the process of sealing by tightening the bonnet 255 to reduce the inside diameter value of the through hole 272 of the elastic gasket 27 to be 0 is omitted.

In this implementation, the inner flanges 2212 arranged on the inner circumference wall of the axial through hole 220 of the spool 22 can be omitted.

The above is the implementation manners of the implementations of the disclosure. It should be pointed out that those of ordinary skill in the art may also make several improvements and modifications without departing from the principle of the implementations of the disclosure. These improvements and modifications shall fall within the scope of protection of the disclosure.

## Claims

1. A hemostatic valve, comprising:
a valve body; and
a spool arranged in the valve body, wherein the spool comprises a spool main body and a cover body connected to a distal end of the spool main body, the spool main body defines an axial through hole therein, and the cover body is operable to be opened or automatically closed relative to the spool main body to correspondingly deocclude or occlude the axial through hole.

2. The hemostatic valve of claim 1, wherein
the spool further comprises an elastic connecting part, the cover body is connected to, at a position close to the axial through hole, the distal end of the spool main body through the elastic connecting part; and
the connecting part forces the cover body to automatically occlude the axial through hole in a natural state.

3. The hemostatic valve of claim 2, wherein the elastic connecting part is an elastic connecting piece connected between the cover body and the spool main body and extending along a periphery of the axial through hole.

4. The hemostatic valve of claim 2, wherein the elastic connecting part is a plurality of elastic connecting rods connected between the cover body and the spool main body and arranged at intervals long a periphery of the axial through hole.

5. The hemostatic valve of claim 1, wherein one of the distal end of the spool main body or a proximal end of the cover body is provided with a first spigot structure, the other is provided with a second spigot structure matching the first spigot structure, and the first spigot structure and the second spigot structure are mutually jointed to form a spigot.

6. The hemostatic valve of claim 5, wherein
the distal end of the spool main body defines a counter bore that is coaxial with the axial through hole and serves as the first spigot structure, a diameter of the counter bore is greater than that of the axial through hole and less than an outside diameter of the cover body;
the proximal end of the cover body is provided with a circular flange that matches the counter bore and serves as the second spigot structure; and
the circular flange is used to be embedded in the counter bore to form a spigot.

7. The hemostatic valve of claim 1, wherein a plurality of inner flanges are axially arranged at intervals on an inner circumference wall of the axial through hole, and each of the plurality of inner flanges is circumferentially arranged in a circle along the inner circumference wall of the axial through hole.

8. The hemostatic valve of claim 7, wherein each two adjacent inner flanges cooperate to encircle a circular groove therebetween, and each of the plurality of inner flanges is used to deform elastically, when being squeezed, to be received in the corresponding circular grooves.

9. The hemostatic valve of claim 1, wherein the cover body is provided with a plurality of reinforcing ribs on at least one of a distal end and a proximal end of the cover body.

10. The hemostatic valve of claim 9, wherein the plurality of reinforcing ribs are uniformly distributed in cross respectively through a center of the cover body or crisscross distributed.

11. The hemostatic valve of claim 1, wherein
the valve body defines a cavity extending through a proximal surface and a distal surface thereof and having an accommodating space; and
the accommodating space has a positioning section and an avoiding section which are arranged coaxially sequentially, wherein the positioning section is closer to a proximal end of the cover body than the avoiding section, the positioning section is used to position the spool main body, and the avoiding section is used to provide a space for opening of the cover body.

12. The hemostatic valve of claim 11, wherein one of an outer circumference surface of the spool main body or the positioning section of the accommodating space is provided with a positioning clamp ring, and the other defines a positioning clamp groove thereon, and the positioning clamp ring is clamped into and in radial interference fit with the positioning clamp groove.

13. The hemostatic valve of claim 12, wherein the positioning clamp ring is in interference fit with the clamp groove in a radial direction of the cavity.

14. The hemostatic valve of claim 11, wherein an outer circumference surface of the valve body hermetically is in close contact with an inner circumference surface of the positioning section of the accommodating space.

15. The hemostatic valve of claim 11, wherein the valve body comprises a valve housing and a bonnet connected to a proximal end of the valve housing, and the accommodating space is defined at one end of the valve housing close to the bonnet.

16. The hemostatic valve of claim 15, wherein
the hemostatic valve further comprises an elastic gasket sandwiched between the spool and the bonnet and defining a through hole communicated to the axial through hole of the spool main body; and
the bonnet is driven to axially move to a distal end or the proximal end to clamp or release the elastic gasket, so that the elastic gasket is allowed to deform to control decrease or increase of a diameter of the through hole thereof.

17. A sheath, comprising a tube body extended with an axial length and the hemostatic valve of any of claims 1 to 16, and the hemostatic valve is arranged at or close to a proximal end of the tube body.

18. The sheath of claim 17, further comprising a handle arranged at the proximal end of the tube body, and the hemostatic valve is arranged at a proximal end or a distal end of the handle.

19. The sheath of claim 17, wherein the tube body is bendable or non-bendable.

20. A catheter sheath assembly, comprising a dilator and the sheath of any of claims 17 to 19, wherein the dilator is movably inserted in the tube body and the axial through hole of the spool main body, the axial through hole occluded by the cover body is deoccluded when the dilator pushes the cover body of the spool main body along a direction toward a distal end, and the cover body automatically resets to occlude the axial through hole when the dilator is withdrawn.
